# EUROPÄISCHE PATENTANMELDUNG

(11) **EP 1 834 941 A2**
(43) Veröffentlichungstag der Anmeldung: **19.09.2007**
(21) Anmeldenummer: 06127302.5
(22) Anmeldetag: 28.12.2006
(51) Int. Cl.: C07C 7/20, C09K 15/30

(54) **Polymerisationsinhibitor zur Stabilisierung von olefinisch ungesättigten Monomeren**

(30) Priorität: 03.03.2006 DE 102006010347
(71) Anmelder: Degussa GmbH, 40474 Düsseldorf (DE)
(72) Erfinder: Meyer, Oliver, 48145, Münster (DE); James, Phillip, Romsey, Hampshire, SO51 8DT (GB); Erpeldinger, Oliver, 42489, Wülfrath (DE); Kraushaar, Frank, 45239, Essen (DE)

(57) **Zusammenfassung**

Die Erfindung betrifft die Verwendung eines Polymerisationsinhibitors zur Stabilisierung von olefinisch ungesättigten Monomeren, die sich dadurch auszeichnet, dass der Polymerisationsinhibitor
• 0 bis 100 Gew.-% einer Verbindung gemäß der Formel (1) 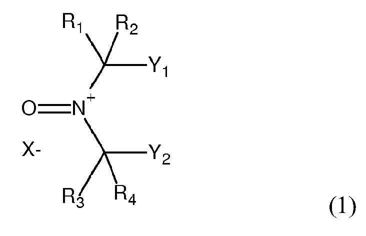 und
• 100 bis 0 Gew.-% einer Verbindung gemäß der Formel (2) 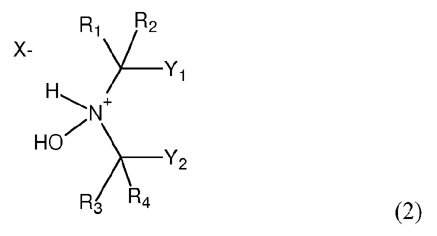 aufweist,
sowie eine Monomerzusammensetzung, die sich dadurch auszeichnet, dass die Monomerzusammensetzung einen Polymerisationsinhibitor, der
• 0 bis 100 Gew.-% einer Verbindung gemäß der Formel (1) und
• 100 bis 0 Gew.-% einer Verbindung gemäß der Formel (2)
aufweist, und zumindest ein olefinisch ungesättigtes Monomer aufweist.

## Beschreibung

Die Erfindung betrifft die Verwendung eines Polymerisationsinhibitors zur Stabilisierung von olefinisch ungesättigten Monomeren sowie eine Monomerzusammensetzung, die sowohl olefinisch ungesättigte Monomere als auch diesen Polymerisationsinhibitor aufweist.

Während der Herstellung von olefinisch ungesättigten Monomeren, wie beispielsweise Ethen, Butadien, Vinylacetat, (Meth)Acrylsäure, (Meth)Acrylate, Acrylnitril oder Styrol, werden diese olefinisch ungesättigten Monomere einem Reinigungsverfahrensschritt unterzogen, wie beispielsweise Destillation oder Extraktion, um unerwünschte Nebenprodukte oder Verunreinigungen zu entfernen.

Bereits während des Herstellungs- und/oder Reinigungsverfahrens können diese olefinisch ungesättigten Monomere polymerisieren. Einige dieser olefinisch ungesättigten Monomere, wie beispielsweise Butadien, neigen bereits bei der Lagerung oder beim Transport zu einer spontanen Polymerisation.

Diese vorzeitige und unerwünschte Polymerisation dieser olefinisch ungesättigten Monomere führt zum einen zu einer Mengenreduktion an einsetzbaren olefinisch ungesättigten Monomeren zum anderen zu einer unerwünschten Abscheidung an unerwünschtem Polymer. Diese Abscheidung des unerwünschten Polymers kann unter Umständen zu einem verminderten Wärmeübergang in einzelnen Anlagenbauteilen führen. Des Weiteren können Oberflächen, die mit dem unerwünschten Polymer überzogen sind, oder Anlagenbauteile, wie beispielsweise Filter, die durch das unerwünschte Polymer verstopft sind, zu einer Unterbrechung der Produktion führen.

Folglich werden den olefinisch ungesättigten Monomeren in der Regel bereits beim Herstellungsverfahren Additive zugesetzt, die entweder als Polymerisationsinhibitoren oder auch als -verzögerer bezeichnet werden, die in der Lage sind, den ungewünschten Prozess der Polymerisation entweder zu unterbinden oder ihn zumindest hinauszuzögern.

Eine Vielzahl an Polymerisationsinhibitoren sind für olefinisch ungesättigte Monomere, wie beispielsweise Styrol bekannt. Unter anderem werden hierfür Verbindungen, wie beispielsweise Schwefel, p-Benzochinon, 4-*tert*.-Butylbrenzcatechin, Phenothiazin oder sterisch gehinderte Phenole, eingesetzt. Einige dieser Verbindungen weisen jedoch beträchtliche Nachteile auf, wie beispielsweise ihre Toxizität, Instabilität bei höheren Temperaturen oder eine unzureichende Wirksamkeit unter den entsprechenden Verfahrensbedingungen des Herstellungs- bzw. Reinigungsverfahrens. Einige der genannten Polymerisationsinhibitoren benötigen sogar Sauerstoff, um ihre Wirkung entfalten zu können, was für die Anwendung in großtechnischen Verfahren zu erheblichen Problemen bzgl. Explosionsschutz führen kann.

Häufig in der Literatur beschriebene Polymerisationsinhibitoren sind sogenannte stabile freie Nitroxylradikale, wie beispielsweise 2,2,6,6-Tetramethylpiperidin-N-oxyl (häufig abgekürzt als TEMPO). So beschreibt beispielsweise die US 3,747,988 die Zugabe eines Nitroxylradikals, insbesondere von 2,2,6,6-Tetramethyl-4-hydroxypiperidin-N-oxyl zum Acrylnitril vor der Destillation. Diese stabilen freien Radikale werden auch in der US 3,488,338 beschrieben hierin allerdings als Kettenabbruchmittel für 2-Chlor-1,3-butadien. Die US 4,670,131 beschreibt den Einsatz dieser stabilen freien Nitroxylradikale als Polymerisationsinhibitor zur Stabilisierung von olefinisch ungesättigten Monomeren, wie beispielsweise Ethen, Propen, Buten oder Butadien.

Den Einsatz von stabilen freien Nitroxylradikalen beschreiben u.a. auch die folgenden Veröffentlichungen "Inhibition of Radical Polymerization by Nitroxide Mono- and Biradicals" (Vysokomol, Soyed. 8: 1966, No. 9, 1642 - 1646) von L. V. Ruban et al., "A Re-Examination of Some Stabilized Radicals as Inhibitors of Polymerization" (J. Polym. Mater. 19 (2002), 113 - 120) und "Stabilized Radicals as Inhibitors of Polymerization - Reactions of Alkoxyamines with Growing Polymer Radicals" (J. Macrom. Science 2002, Vol. A39, No. 11, 1295 - 1303) von Bevington et al..

In zahlreichen Veröffentlichungen wird der Einsatz von Zusammensetzungen, die u.a. stabile freie Nitroxylradikale aufweisen, beschrieben. So beschreiben US 6,525,146, WO 2002/088055 und EP 1 077 245 eine Zusammensetzung aus stabilen freien Nitroxylradikalen und Phenolderivaten als Polymerisationsinhibitor. Weitere Beispiele einer Zusammensetzung für die Stabilisierung von olefinisch ungesättigten Monomere, die stabile freie Nitroxylradikale aufweisen, beschreiben beispielsweise auch US 2003/080318, WO 2002/094884, WO 2002/033026, WO 2002/00816, EP 1 077 206 und DE 199 56 509.

Es war deshalb die Aufgabe der vorliegenden Erfindung einen Polymerisationsinhibitor für olefinisch ungesättigte Monomere mit einer gegenüber dem Stand der Technik verbesserten Wirkung bereit zu stellen. Insbesondere sollte ein Polymerisationsinhibitor bereit gestellt werden, der verbesserte Eigenschaften bei der Stabilisierung von olefinisch ungesättigten Monomeren gegenüber vorzeitiger Polymerisation während des Herstellungs-, Reinigungsverfahren oder während der Lagerung aufweist.

Überraschenderweise wurde gefunden, dass Verbindungen gemäß den Formeln (1) und/oder (2) als Polymerisationsinhibitoren für olefinisch ungesättigte Monomere geeignet sind. Die Herstellung dieser Verbindungen ist seit längerer Zeit bekannt, es wurde jedoch nicht erkannt, dass diese Verbindungen als Polymerisationsinhibitoren eingesetzt werden können. Ferner war es überraschend, dass diese Verbindungen, die über kein stabiles freies Radikal verfügen, diese Wirkung als Polymerisationsinhibitor entfalten können. Die Lösung dieser Aufgabe war umso überraschender, zumal sich zeigte, dass die Wirkung dieser Verbindungen als Polymerisationsinhibitoren eine gegenüber Polymerisationsinhibitoren gemäß dem Stand der Technik, die ein stabiles freies Radikal in ihrer Struktur aufweisen, verbessert ist. Bei gleicher molarer Menge an Polymerisationsinhibitor in den olefinisch ungesättigten Monomeren kann im Vergleich zum Stand der Technik gemäß der erfindungsgemäßen Verwendung ein geringerer Polymeranteil in den olefinisch ungesättigten Monomeren erhalten werden. Beziehungsweise kann der gleiche Polymeranteil in den olefinisch ungesättigten Monomeren durch eine geringere molare Menge an Polymerisationsinhibitor erzielt werden.

Gegenstand der Erfindung ist die Verwendung eines Polymerisationsinhibitors zur Stabilisierung von olefinisch ungesättigte Monomeren, die sich dadurch auszeichnet, dass der Polymerisationsinhibitor
- 0 bis 100 Gew.-% einer Verbindung gemäß der Formel (1) 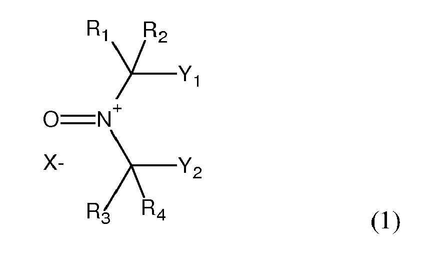 und
- 100 bis 0 Gew.-% einer Verbindung gemäß der Formel (2) 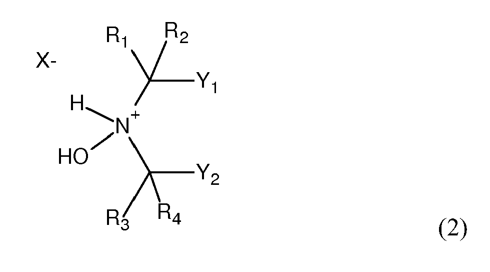 mit:
   - R₁, R₂, R₃ und R₄ =: Alkylgruppe mit 1 bis 4 Kohlenstoffatomen und
   - Y₁, Y₂ =: Alkylgruppe mit 1 bis 4 Kohlenstoffatomen oder Y₁ und Y₂ zusammen ein Ringsystem bilden,
   - X⁻ =: anorganisches oder organisches Anion
   wobei die Substituenten vom Typ R₁, R₂, R₃, R₄, Y₁ und Y₂ gleich oder unterschiedlich sind, sowohl die Substituenten vom Typ Y₁ und Y₂ als auch das auf den Substituenten vom Typ Y₁ und Y₂ basierende Ringsystem unsubstituiert oder substituiert vorliegen und die Summe der Gewichtsprozente der Verbindungen gemäß der Formel (1) und (2) 100 Gew.-% ergibt,
aufweist, mit einem olefinisch ungesättigten Monomer oder Monomermischung, die zumindest ein olefinisch ungesättigtes Monomer aufweist, vermischt wird.

Weiterer Gegenstand dieser Erfindung ist eine Monomerzusammensetzung, die sich dadurch auszeichnet, dass die Monomerzusammensetzung einen Polymerisationsinhibitor, der
- 0 bis 100 Gew.-% einer Verbindung gemäß der Formel (1) und
- 100 bis 0 Gew.-% einer Verbindung gemäß der Formel (2)
aufweist, und zumindest ein olefinisch ungesättigtes Monomer aufweist.

Die erfindungsgemäße Verwendung eines Polymerisationsinhibitors zur Stabilisierung von olefinisch ungesättigte Monomere, zeichnet sich dadurch aus, dass der Polymerisationsinhibitor
- 0 bis 100 Gew.-% einer Verbindung gemäß der Formel (1) 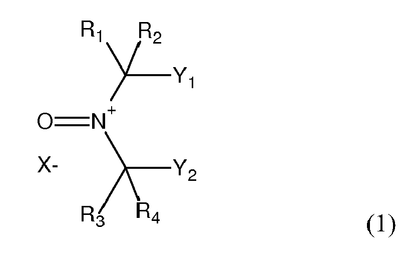 und
- 100 bis 0 Gew.-% einer Verbindung gemäß der Formel (2) 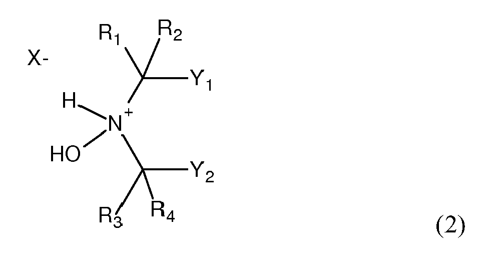 mit:
   - R₁, R₂, R₃ und R₄ =: Alkylgruppe mit 1 bis 4 Kohlenstoffatomen und
   - Y₁, Y₂ =: Alkylgruppe mit 1 bis 4 Kohlenstoffatomen oder Y₁ und Y₂ zusammen ein Ringsystem bilden,
   - X⁻ =: anorganisches oder organisches Anion
   wobei die Substituenten vom Typ R₁, R₂, R₃, R₄, Y₁ und Y₂ gleich oder unterschiedlich sind, sowohl die Substituenten vom Typ Y₁ und Y₂ als auch das auf den Substituenten vom Typ Y₁ und Y₂ basierende Ringsystem unsubstituiert oder substituiert vorliegen und die Summe der Gewichtsprozente der Verbindungen gemäß der Formel (1) und (2) 100 Gew.-% ergibt,
aufweist, mit einem olefinisch ungesättigten Monomer oder Monomermischung, die zumindest ein olefinisch ungesättigtes Monomer aufweist, vermischt wird.

In der erfindungsgemäßen Verwendung dieser Polymerisationsinhibitoren können entweder Verbindungen gemäß der Formel (1) oder gemäß der Formel (2) eingesetzt werden. Bevorzugt wird in der erfindungsgemäßen Verwendung jedoch eine Zusammensetzung als Polymerisationsinhibitor eingesetzt, die sowohl Verbindungen gemäß der Formel (1) als auch der Formel (2) aufweist. Insbesondere wird ein Polymerisationsinhibitor eingesetzt, der
- 40 bis 60 Gew.-% einer Verbindung gemäß der Formel (1)
und
- 60 bis 40 Gew.-% einer Verbindung gemäß der Formel (2)
aufweist. Besonders bevorzugt wird ein Polymerisationsinhibitor eingesetzt, der
- 45 bis 55 Gew.-% einer Verbindung gemäß der Formel (1)
und
- 55 bis 45 Gew.-% einer Verbindung gemäß der Formel (2)
aufweist.

Die in der erfindungsgemäßen Verwendung eingesetzten Polymerisationsinhibitoren können gemäß einem Verfahren hergestellt werden, wie es Merbouh et al. in "Preparation of tetramethylpiperidine-1-oxoammonium salts and their use as oxidants in organic chemistry. A review" (Organic Preparations and Procedures International, 2004, 36 (1), 3-31) oder Golubev et al. in "Some reactions of free iminoxyl radicals with the participation of the unpaired electron" (Seriya Khimicheskaya, No. 11, 1965, 1927-1936) beschreiben.

Bei einem bevorzugten Verfahren zur Herstellung dieser Verbindungen gemäß Formel (1) entstehen in der Regel sowohl Verbindungen gemäß der Formel (1) als auch der Formel (2), hierbei wird das entsprechende Nitroxylradikal in Lösung mit einer starken anorganischen oder organischen Säure umgesetzt. Durch die Disproportionierung des Nitroxylradikals entsteht ein ca. 1:1-Gemisch des entsprechenden Oxoammoniumsalzes gemäß der Formel (1) und der entsprechenden Hydroxylaminverbindung gemäß der Formel (2). Setzt man in der erfindungsgemäßen Verwendung daher eine Zusammensetzung dieser Verbindungen gemäß Formel (1) und (2) ein, so kann eine aufwändige Trennung der beiden Verbindungen nach dem Herstellungsprozess entfallen.

In einer besonderen Ausführungsform der erfindungsgemäßen Verwendung dieser Polymerisationsinhibitoren werden diese in-situ hergestellt.

In einer besonderen Ausführungsform der erfindungsgemäßen Verwendung sind die Substituenten vom Typ Y₁ und Y₂ substituiert, wobei der Substituent oder diese Substituenten der Substituenten vom Typ Y₁ und Y₂ ausgewählt sind aus, Alkyl-, Ester-, Ether-, Hydroxy-, Oxo-, Cyan-, Cyanhydrin-, Amino-, Amid-, Carboxy-, Halogen-, Hydantoin-, Ketal-, Acetal- oder Urethangruppe.

Vorzugsweise bilden die beiden Substituenten vom Typ Y₁ und Y₂ des Polymerisationsinhibitors zusammen ein Ringsystem aus. Besonders bevorzugt wird in der erfindungsgemäßen Verwendung ein Polymerisationsinhibitor eingesetzt, der
- 0 bis 100 Gew.-% einer Verbindung gemäß der Formel (3) 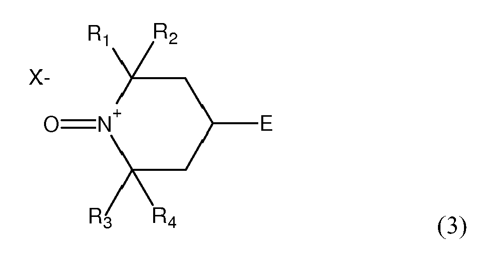 und
- 100 bis 0 Gew.-% einer Verbindung gemäß der Formel (4) 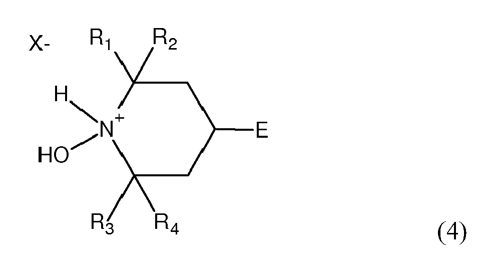 mit:
   - R₁, R₂, R₃ und R₄ =: Alkylgruppe mit 1 bis 4 Kohlenstoffatomen,
   - E =: Alkyl-, Ester-, Ether-, Hydroxy-, Oxo-, Cyan-, Cyanhydrin-, Amino-, Amid-, Carboxy-, Halogen-, Hydantoin-, Ketal-, Acetal- oder Urethangruppe und
   - X⁻ =: anorganisches oder organisches Anion,
wobei die Substituenten vom Typ R₁, R₂, R₃ und R₄ gleich oder unterschiedlich sind, aufweist. Ganz besonders bevorzugt wird in der erfindungsgemäßen Verwendung ein Polymerisationsinhibitor eingesetzt, der Verbindungen gemäß der Formel (3) und / oder (4) mit R₁, R₂, R₃ und R₄ = Methylgruppe aufweist.

Insbesondere werden in der erfindungsgemäßen Verwendung Polymerisationsinhibitoren eingesetzt, die ein anorganisches oder ein organisches Anion in den Verbindungen gemäß den Formeln (1) bis (4) aufweisen, bevorzugt weisen die eingesetzten Polymerisationsinhibitoren anorganische Anionen auf. Besonderes bevorzugt werden in der erfindungsgemäßen Verwendung Polymerisationsinhibitoren eingesetzt, die als Anion X⁻ in den Verbindungen gemäß den Formeln (1) bis (4) ein Halogenanion, bevorzugt Chlor- oder Bromanion, aufweisen.

Im Rahmen dieser Erfindung wird unter einem Polymerisationsinhibitor eine Verbindung verstanden, die in der Lage ist, eine Polymerisation des olefinisch ungesättigten Monomers für eine gewisse Zeitdauer zu unterbinden. Die Zeitdauer - bis bei einem olefinisch ungesättigten Monomer ohne einen Polymerisationsinhibitor die Polymerisation eintritt - ist daher kürzer als die Zeitdauer bei einem olefinisch ungesättigten Monomers mit einem Polymerisationsinhibitor.

Ferner werden im Rahmen dieser Erfindung unter olefinisch ungesättigten Monomeren Verbindungen verstanden, die zumindest eine C-C-Doppelbindung aufweisen und in der Lage sind eine Polymerisationsreaktion einzugehen.

In der erfindungsgemäßen Verwendung des Polymerisationsinhibitors werden vorzugsweise zumindest ein olefinisch ungesättigtes Monomer, ausgewählt aus Alk-1-enen oder Alka-1,3-dienen, die sowohl substituiert als auch unsubstituiert sein können, eingesetzt. Bevorzugt werden olefinisch ungesättigte Monomere, ausgewählt aus Ethen, Propen bzw. Propylen, Butadien, Vinylacetat, (Meth)Acrylsäure, (Meth)Acrylat, Acrylnitril, Acrolein, N-Vinylformamid, Chloropren, Isopren, Divinylbenzol oder Styrol, eingesetzt.

Im Rahmen dieser Erfindung werden unter (Meth)Acrylsäure sowohl Acrylsäure als auch Methacrylsäure verstanden und unter (Meth)Acrylat sowohl Acrylsäureester als auch Methacrylsäureester, wobei diese olefinisch ungesättigten Monomere substituiert oder unsubstituiert sein können.

In der erfindungsgemäßen Verwendung kann sowohl eine Verbindung an olefinisch ungesättigten Monomeren als auch eine Mischung an verschiedenen olefinisch ungesättigten Monomeren eingesetzt werden.

Bei der erfindungsgemäßen Verwendung können die Polymerisationsinhibitoren als Feststoff, Lösung oder als Suspension den olefinisch ungesättigten Monomeren zugesetzt werden. Die Polymerisationsinhibitoren können hierbei auch während eines Verfahrens, wie beispielsweise Herstellungs- oder Reinigungsverfahrens, den olefinisch ungesättigten Monomeren zugesetzt werden. Vorteilhaft ist es, bei der erfindungsgemäßen Verwendung dieser Polymerisationsinhibitoren auf ein geeignetes Löse- bzw. Dispersionsmittel zu achten, das zum einen mit dem olefinisch ungesättigten Monomer als auch mit diesen Polymerisationsinhibitoren verträglich ist und es nicht zu ungewünschten Reaktionen kommen kann.

Diese Polymerisationsinhibitoren können nach gängigen Methoden des Stands der Technik den ungesättigten Monomeren bzw. Monomermischungen zugegeben werden. Vorteilhafterweise können bei der erfindungsgemäßen Verwendung die Polymerisationsinhibitoren in den Zulaufstrom (feed-stream) einer Destillationskolonne, in die Zu- und Ableitung eines Wärmetauschers oder eines Verdampfers ("Autkochers") oder in die Zu- und Ableitung eines Kondensators zugegeben werden. Darüber hinaus können in der erfindungsgemäßen Verwendung die Polymerisationsinhibitoren auch in Lagertanks der olefinisch ungesättigten Monomere hinzugefügt werden.

Unter dem Begriff "effektive Menge an dem erfindungsgemäßen Polymerisationsinhibitor" wird im Rahmen dieser Erfindung die Menge an Polymerisationsinhibitor verstanden, die notwendig ist, um die vorzeitige Polymerisation der olefinisch ungesättigten Monomere zu verhindern. Diese effektive Menge an dem Polymerisationsinhibitor ist abhängig von den Bedingungen, unter denen das olefinisch ungesättigte Monomer gelagert oder gehandhabt wird. Beispielsweise ist bei der Destillation des ungesättigten Monomers auf Grund der höheren Temperaturen und der höheren Konzentration an Verunreinigungen eine höhere Menge an dem Polymerisationsinhibitor notwendig.

Bevorzugt werden bei der erfindungsgemäßen Verwendung des Polymerisationsinhibitors dem olefinisch ungesättigten Monomer oder dem Monomergemisch von 100 ppb (m/m) bis 10000 ppm (m/m), besonders bevorzugt von 1 ppm (m/m) bis 1000 ppm (m/m) und ganz besonders bevorzugt von 10 ppm (m/m) bis 100 ppm (m/m) an Polymerisationsinhibitor bezogen auf das olefinisch ungesättigte Monomer zugemischt.

Die erfindungsgemäße Monomerzusammensetzung zeichnet sich dadurch aus, dass die Monomerzusammensetzung einen Polymerisationsinhibitor, der
- 0 bis 100 Gew.-% einer Verbindung gemäß der Formel (1) 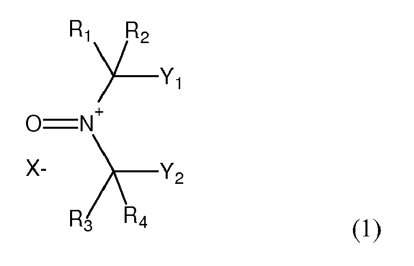 und
- 100 bis 0 Gew.-% einer Verbindung gemäß der Formel (2) 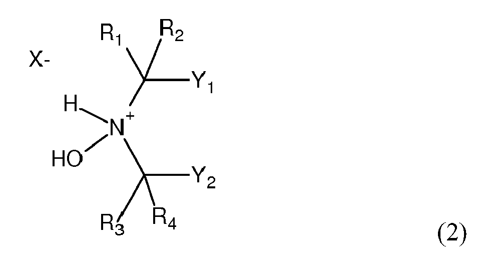 mit:
   - R₁, R₂, R₃ und R₄ =: Alkylgruppe mit 1 bis 4 Kohlenstoffatomen und
   - Y₁, Y₂ =: Alkylgruppe mit 1 bis 4 Kohlenstoffatomen oder Y₁ und Y₂ zusammen ein Ringsystem bilden,
   - X⁻ =: anorganisches oder organisches Anion
   wobei die Substituenten vom Typ R₁, R₂, R₃, R₄, Y₁ und Y₂ gleich oder unterschiedlich sind, sowohl die Substituenten vom Typ Y₁ und Y₂ als auch das auf den Substituenten vom Typ Y₁ und Y₂ basierende Ringsystem unsubstituiert oder substituiert vorliegen und die Summe der Gewichtsprozente der Verbindungen gemäß der Formel (1) und (2) 100 Gew.-% ergibt,
aufweist, und zumindest ein olefinisch ungesättigtes Monomer aufweist.

Die erfindungsgemäße Monomerzusammensetzung kann entweder Verbindungen gemäß der Formel (1) oder gemäß der Formel (2) als Polymerisationsinhibitor aufweisen. Bevorzugt weist die erfindungsgemäße Monomerzusammensetzung sowohl Verbindungen gemäß der Formel (1) als auch der Formel (2) als Polymerisationsinhibitor auf. Insbesondere weist die erfindungsgemäße Monomerzusammensetzung einen Polymerisationsinhibitor auf, der
- 40 bis 60 Gew.-% einer Verbindung gemäß der Formel (1)
   und
- 60 bis 40 Gew.-% einer Verbindung gemäß der Formel (2)
aufweist. Besonders bevorzugt weist die Monomerzusammensetzung einen Polymerisationsinhibitor auf, der
- 45 bis 55 Gew.-% einer Verbindung gemäß der Formel (1)
   und
- 55 bis 45 Gew.-% einer Verbindung gemäß der Formel (2) aufweist.

In einer besonderen Ausführungsform der erfindungsgemäßen Monomerzusammensetzung sind die Substituenten vom Typ Y₁ und Y₂ bei den Verbindungen gemäß den Formeln (1) und (2) des Polymerisationsinhibitors substituiert, wobei der Substituent oder diese Substituenten der Substituenten vom Typ Y₁ und Y₂ ausgewählt sind aus, Alkyl-, Ester-, Ether-, Hydroxy-, Oxo-, Cyan-, Cyanhydrin-, Amino-, Amid-, Carboxy-, Halogen-, Hydantoin-, Ketal-, Acetal- oder Urethangruppe.

Vorzugsweise bilden die beiden Substituenten vom Typ Y₁ und Y₂ der Verbindungen gemäß den Formeln (1) und (2) des Polymerisationsinhibitors zusammen ein Ringsystem aus. Besonders bevorzugt weist die erfindungsgemäße Monomerzusammensetzung einen Polymerisationsinhibitor auf, der
- 0 bis 100 Gew.-% einer Verbindung gemäß der Formel (3) 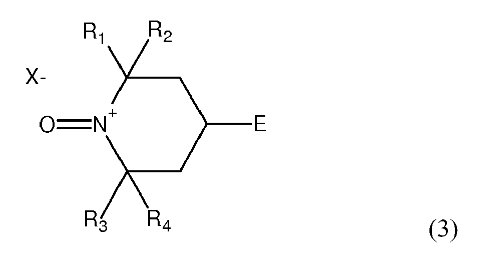 und
- 100 bis 0 Gew.-% einer Verbindung gemäß der Formel (4) 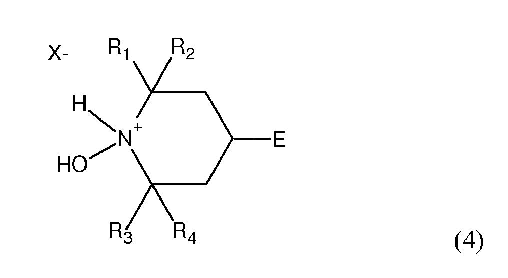 mit:
   - R₁, R₂, R₃ und R₄ =: Alkylgruppe mit 1 bis 4 Kohlenstoffatomen,
   - E =: Alkyl-, Ester-, Ether-, Hydroxy-, Oxo-, Cyan-, Cyanhydrin-, Amino-, Amid-, Carboxy-, Halogen-, Hydantoin-, Ketal-, Acetal- oder Urethangruppe und
   - X⁻ =: anorganisches oder organisches Anion,
wobei die Substituenten vom Typ R₁, R₂, R₃ und R₄ gleich oder unterschiedlich sind, aufweist. Ganz besonders bevorzugt weist die erfindungsgemäße Monomerzusammensetzung Verbindungen gemäß der Formel (3) und / oder (4) mit R₁, R₂, R₃ und R₄ = Methylgruppe als Polymerisationsinhibitor auf.

Insbesondere weist die erfindungsgemäße Monomerzusammensetzung ein anorganisches oder ein organisches Anion in den Verbindungen gemäß den Formeln (1) bis (4) auf, bevorzugt weisen diese anorganische Anionen auf. Besonderes bevorzugt weist die erfindungsgemäße Monomerzusammensetzung als Anion X⁻ in den Verbindungen gemäß den Formeln (1) bis (4) ein Halogenanion, bevorzugt Chlor- oder Bromanion, auf.

Die erfindungsgemäße Monomerzusammensetzung weist vorzugsweise zumindest ein olefinisch ungesättigtes Monomer, ausgewählt aus Alk-1-enen oder Alka-1,3-enen, die sowohl substituiert als auch unsubstituiert sein können, auf. Bevorzugt weist diese olefinisch ungesättigte Monomere, ausgewählt aus Ethen, Propen bzw. Propylen, Butadien, Vinylacetat, (Meth)Acrylsäure, (Meth)Acrylat, Acrylnitril, Acrolein, N-Vinylformamid, Chloropren, Isopren, Divinylbenzol oder Styrol, auf.

Die erfindungsgemäße Monomerzusammensetzung kann sowohl eine Verbindung an olefinisch ungesättigten Monomeren als auch eine Mischung an verschiedenen olefinisch ungesättigten Monomeren aufweisen.

Bevorzugt weisen die erfindungsgemäßen Monomerzusammensetzungen von 100 ppb (m/m) bis 10000 ppm (m/m), besonders bevorzugt von 1 ppm (m/m) bis 1000 ppm (m/m) und ganz besonders bevorzugt von 10 ppm (m/m) bis 100 ppm (m/m) an Polymerisationsinhibitor bezogen auf das olefinisch ungesättigte Monomer auf.

Die nachfolgenden Beispiele sollen die erfindungsgemäße Verwendung näher erläutern, ohne dass die Erfindung auf diese Ausführungsformen beschränkt sein soll.

Herstellung der Polymerisationsinhibitoren:

### Beispiel 1:

Es wurde in Beispiel 1 kein Polymerisationsinhibitor eingesetzt.

### Beispiel 2:

Als Polymerisationsinhibitor wurde in Beispiel 2 kommerziell erhältliches 4-Hydroxy-TEMPO der Fa. Degussa ohne weitere Aufarbeitung eingesetzt.

### Beispiel 3:

Der Polymerisationsinhibitor in Beispiel 3 wurde gemäß einem Verfahren hergestellt, das von Paleos et al. in "Ready Reduction of Some Nitroxide Free Radicals Using Ascorbic Acid" (J. Chem. Soc, Chem. Comm., 1997, 345-346) beschrieben ist.

### Beispiele 4 und 5:

Die Polymerisationsinhibitoren in den Beispielen 4 und 5 wurden gemäß einem Verfahren hergestellt, das Merbouh et al. in dem experimentellen Teil auf den Seiten 24 bis 26 in "Preparation of tetramethylpiperidine-1-oxoammonium salts and their use as oxidants in organic chemistry. A review" (Organic Preparations and Procedures International, 2004, 36 (1), 3-31) oder das Golubev et al. in "Some Reactions Of Free Iminoxyl Radicals With The Participation Of The Unpaired Electron" (Izvestiya Akademii Nauk SSSR, Seriya Khimicheskaya, No. 11, 1965, 1927-1936) beschreiben. Als Edukt wurde das 4-Hydroxy-TEMPO der Fa. Degussa eingesetzt. Bei dem Polymerisationsinhibitor des Beispiels 5 wurde analytisch nachgewiesen, dass es sich um das Monobromid handelt.

### Beispiel 6:

Der in Beispiel 6 eingesetzte Polymerisationsinhibitor wurde hergestellt, indem 1 g 1,4-Dihydroxy-2,2,6,6-tetramethylpiperidin in 200 ml entgastem Dichlorethan gelöst wurden und anschließend bei Raumtemperatur langsam für eine Stunde HCl-Gas in die Lösung eingeleitet wurde. Nach einer Stunde Nachreaktion wurde der ausgefallene gelbe Niederschlag abfiltriert, mit tert.-Butylmethylether gewaschen und getrocknet.

### Beispiele 7 und 8:

Der in den Beispielen 7 und 8 eingesetzte Polymerisationsinhibitor wurde hergestellt, indem 0,05 mol 4-Hydroxy-TEMPO der Fa. Degussa in 200 g Dichlorethan gelöst wurden und anschließend unter leichter Kühlung bei Raumtemperatur langsam für eine Stunde 0,1 mol HCl-Gas in die Lösung eingeleitet wurde. Nach einer Stunde Nachreaktion wurde der ausgefallene gelbe Niederschlag abfiltriert, mit Dichlormethan gewaschen und getrocknet.

### Versuche zur Stabilität der Monomermischung:

Käuflich erwerbbares Styrol (der Fa. Fluka, purum, monomer, ≥ 99,0% (GC)) wird bei einem reduziertem Druck von 95 hPa, einer Sumpftemperatur von 75°C und einer Stickstoffbedeckung von dem Stabilisator tert.-Butyl-1,2-hydroxybenzol befreit. Ein Dreihalskolben mit einem Thermometer, Kühler, Septum und einem Magnetrührer wird gründlich mit Stickstoff gespült, um eine sauerstofffreie Atmosphäre in dem Kolben zu erhalten. Diese Stickstoffatmosphäre wird über die gesamte Versuchsdauer beibehalten. Der Dreihalskolben wird mit genau 300 g des destillierten Styrols und der jeweiligen Menge an Polymerisationsinhibitor befüllt. Um den Versuch zu starten, wird der Dreihalskolben in ein auf 110°C vorgeheiztes Ölbad getaucht, wobei die in dem Dreihalskolben enthaltene stabilisierte Monomerzusammensetzung gerührt wird. Die Menge und die Art des Polymerisationsinhibitors ist der Tabelle 1 zu entnehmen. Nach dem Eintauchen des Dreihalskolbens in das geheizte Ölbad werden in regelmäßigen Abständen 2 g der Monomerzusammensetzung als Probe entnommen, genau ausgewogen und in 10 ml Methanol gegeben. Nach 30 Minuten wird das aus Methanol ausgefällte Polystyrol mittels eines Glasfiltertiegels abgetrennt, für 5 Stunden bei 110°C getrocknet und genau gewogen. Die Menge an vorhandenem Polystyrol in der Probe der Monomerzusammensetzung wird in einem Diagramm gegen die Reaktionszeit aufgetragen. Als Vergleichswert zwischen den Beispielen wird für jeden Polymerisationsinhibitor die Zeitdauer ermittelt bzw. ggf. interpoliert, bei der die Probe der Monomerzusammensetzung laut Messung einen Polymergehalt von 3 Gew.-% erreicht hat.

**Tabelle 1:**

| Beispiel | Polymerisationsinhibitor | aktive Menge an Polymerisationsinhibitor *[in ppm (m*/*m)]* | Zeitdauer, nach der die Probe der Monomerzusammensetzung einen Polymergehalt von 3 Gew.-% erreicht hat. *[in Minuten]* |
|---|---|---|---|
| 1 | | - | 50 |
| 2 | 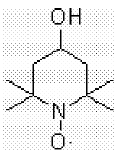 | 100 | 120 |
| 3 | 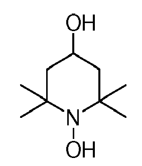 | 100 | 90 |
| 4 | 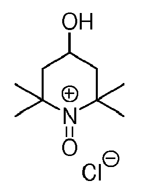 | 100^{*)} | 145 |
| 5 | 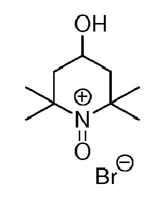 | 100 ^{*)} | 210 |
| 6 | 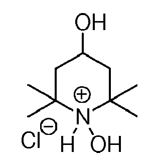 | 100 ^{*)} | 255 |
| 7 | 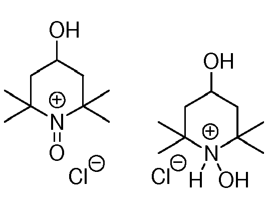 | je 50 (insgesamt 100) ^{*)} | 210 |
| 8 | 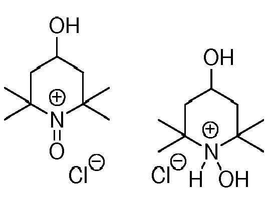 | je 50 (insgesamt 100) ^{**)} | 205 |

| | | | |
|---|---|---|---|
| ^{*)} Bei der Berechnung der Menge an Polymerisationsinhibitoren wurden bei den ionischen Verbindungen, nur das Molekulargewicht des Kations und nicht des Anions berücksichtigt. **) Bei Beispiel 8 wurde im Gegensatz zu Beispiel 7 auch das Molekulargewicht des Anions berücksichtigt. | | | |

Bei den Beispielen 4 - 8 handelt es sich um erfindungsgemäße Beispiele.

## Patentansprüche

1. Verwendung eines Polymerisationsinhibitors zur Stabilisierung von olefinisch ungesättigte Monomeren,
**dadurch gekennzeichnet,**
**dass** der Polymerisationsinhibitor
0 bis 100 Gew.-% einer Verbindung gemäß der Formel (1) 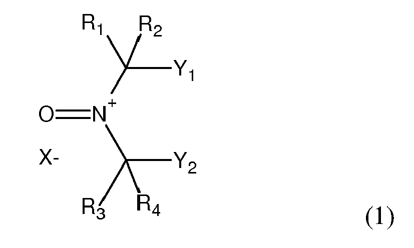 und
100 bis 0 Gew.-% einer Verbindung gemäß der Formel (2) 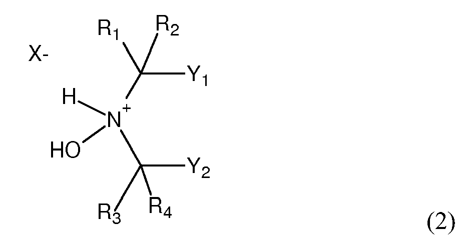 mit:
R₁, R₂, R₃ und R₄ = Alkylgruppe mit 1 bis 4 Kohlenstoffatomen und
Y₁, Y₂ = Alkylgruppe mit 1 bis 4 Kohlenstoffatomen oder Y₁ und Y₂ zusammen ein Ringsystem bilden,
X⁻ = anorganisches oder organisches Anion,
wobei die Substituenten vom Typ R₁, R₂, R₃, R₄, Y₁ und Y₂ gleich oder unterschiedlich sind, sowohl die Substituenten vom Typ Y₁ und Y₂ als auch das auf den Substituenten vom Typ Y₁ und Y₂ basierende Ringsystem unsubstituiert oder substituiert vorliegen und die Summe der Gewichtsprozente der Verbindungen gemäß der Formel (1) und (2) 100 Gew.-% ergibt,
aufweist, mit einem olefinisch ungesättigten Monomer oder Monomermischung, die zumindest ein olefinisch ungesättigtes Monomer aufweist, vermischt wird.

2. Verwendung gemäß Anspruch 1,
**dadurch gekennzeichnet,**
**dass** die Substituenten vom Typ Y₁ und Y₂ substituiert sind, wobei der Substituent oder diese Substituenten der Substituenten vom Typ Y₁ und Y₂ ausgewählt sind aus, Alkyl-, Ester-, Ether-, Hydroxy-, Oxo-, Cyan-, Cyanhydrin-, Amino-, Amid-, Carboxy-, Halogen-, Hydantoin-, Ketal-, Acetal- oder Urethangruppe.

3. Verwendung gemäß Anspruch 1 oder 2,
**dadurch gekennzeichnet,**
**dass** zumindest ein olefinisch ungesättigtes Monomer, ausgewählt aus Ethen, Propen bzw. Propylen, Butadien, Vinylacetat, (Meth)Acrylsäure, (Meth)Acrylat, Acrylnitril, Acrolein, N-Vinylformamid, Chloropren, Isopren, Divinylbenzol oder Styrol, eingesetzt wird.

4. Verwendung gemäß zumindest einem der Ansprüche 1 bis 3,
**dadurch gekennzeichnet,**
**dass** dem Monomer oder dem Monomergemisch von 100 ppb (m/m) bis 10000 ppm (m/m) an Polymerisationsinhibitor bezogen auf das olefinisch ungesättigte Monomer zugemischt wird.

5. Verwendung gemäß zumindest einem der Ansprüche 1 bis 4,
**dadurch gekennzeichnet,**
**dass** dem Monomer oder dem Monomergemisch von 1 ppm (m/m) bis 1000 ppm (m/m) an Polymerisationsinhibitor bezogen auf das olefinisch ungesättigte Monomer zugemischt wird.

6. Verwendung gemäß zumindest einem der Ansprüche 1 bis 5,
**dadurch gekennzeichnet,**
**dass** ein Polymerisationsinhibitor eingesetzt wird, der
0 bis 100 Gew.-% einer Verbindung gemäß der Formel (3) 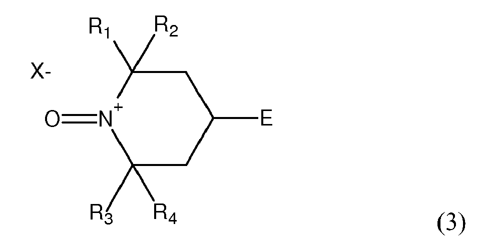 und
100 bis 0 Gew.-% einer Verbindung gemäß der Formel (4) 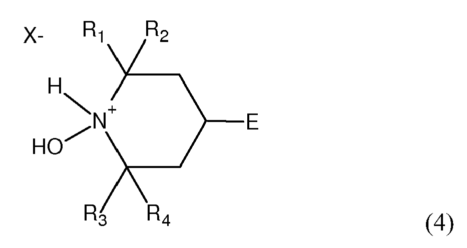 mit:
R₁, R₂, R₃ und R₄ = Alkylgruppe mit 1 bis 4 Kohlenstoffatomen und
E = Alkyl-, Ester-, Ether-, Hydroxy-, Oxo-, Cyan-, Cyanhydrin-, Amino-, Amid-, Carboxy-, Halogen-, Hydantoin-, Ketal-, Acetal- oder Urethangruppe und
X⁻ = anorganisches oder organisches Anion,
wobei die Substituenten vom Typ R₁, R₂, R₃ und R₄ gleich oder unterschiedlich sind, aufweist.

7. Monomerzusammensetzung,
**dadurch gekennzeichnet,**
**dass** die Monomerzusammensetzung einen Polymerisationsinhibitor, der
0 bis 100 Gew.-% einer Verbindung gemäß der Formel (1) 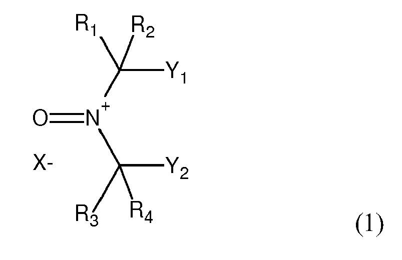 und
100 bis 0 Gew.-% einer Verbindung gemäß der Formel (2) 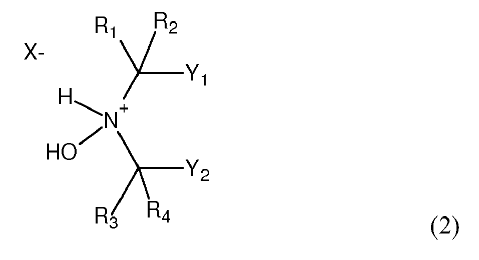 mit:
R₁, R₂, R₃ und R₄ = Alkylgruppe mit 1 bis 4 Kohlenstoffatomen und
Y₁, Y₂ = Alkylgruppe mit 1 bis 4 Kohlenstoffatomen oder Y₁ und Y₂ zusammen ein Ringsystem bilden,
X⁻ = anorganisches oder organisches Anion,
wobei die Substituenten vom Typ R₁, R₂, R₃, R₄, Y₁ und Y₂ gleich oder unterschiedlich sind, sowohl die Substituenten vom Typ Y₁ und Y₂ als auch das auf den Substituenten vom Typ Y₁ und Y₂ basierende Ringsystem unsubstituiert oder substituiert vorliegen und die Summe der Gewichtsprozente der Verbindungen gemäß der Formel (1) und (2) 100 Gew.-% ergibt,
aufweist, und zumindest ein olefinisch ungesättigtes Monomer aufweist.
